# EUROPEAN PATENT APPLICATION

(11) **EP 4 420 605 A2**
(43) Date of publication of application: **28.08.2024**
(21) Application number: 24156801.3
(22) Date of filing: 09.02.2024
(51) Int. Cl.: A61B 5/021, A61B 5/024, A61B 5/11, A61B 5/1455, A61B 5/296, A61B 5/389, A61B 5/08

(54) **USE OF ELECTROMYOGRAPHY (EMG) ELECTRODES FOR PHYSIOLOGICAL MEASUREMENTS**

(30) Priority: 22.02.2023 US 202363486366 P; 24.01.2024 US 202418421543
(71) Applicant: Meta Platforms Technologies, LLC, Menlo Park, CA 94025 (US)
(72) Inventor: Shaga, Ravi Krishna, Menlo Park (US)
(74) Representative: Murgitroyd & Company

(57) **Abstract**

Disclosed herein are methods, devices, and media for determining physiological characteristics. In some embodiments, a method involves obtaining an electromyography (EMG) signal representing muscle activity from an EMG electrode disposed in or on a band of a wrist-worn device, and a photoplethysmography (PPG) signal using a PPG sensor of the wrist-worn device. The method may involve generating a modified PPG signal using the EMG signal, wherein the modified PPG signal corrects for motion artifacts in the PPG signal due to motion activity of a wearer of the wrist-worn device. The method may involve determining at least one physiological characteristic based on the modified PPG signal.

## Description

### CROSS-REFERENCE TO RELATED APPLICATIONS

This application claims priority under 35 U.S.C. §119 to U.S. Provisional Application 63/486,366, filed on February 22, 2023.

### BACKGROUND

Wearable user devices, such as smart watches, fitness trackers, wrist-bands, etc., may be used to measure various physiological characteristics of a wearer of the device. These physiological characteristics may include heart rate, oxygen saturation, respiration rate, blood pressure, etc. Because these physiological characteristics are linked to user health, accuracy of these measurements is important. However, it may be difficult to accurately measure certain physiological characteristics, which may in turn cause harm to the wearer.

### SUMMARY

According to a first aspect of the present invention there is provided a method of determining physiological characteristics, the method comprising: obtaining an electromyography (EMG) signal representing muscle activity from an EMG electrode disposed in or on a band of a wrist-worn device, and a photoplethysmography (PPG) signal using a PPG sensor of the wrist-worn device; generating a modified PPG signal using the EMG signal, wherein the modified PPG signal corrects for motion artifacts in the PPG signal due to motion activity of a wearer of the wrist-worn device; and determining at least one physiological characteristic based on the modified PPG signal.

Optionally, the at least one physiological characteristic is at least one of: a heart rate of the wearer, a blood pressure of the wearer, or an oxygen saturation of the wearer.

Optionally, the muscle activity comprises activity from at least one wrist extensor muscle or at least one wrist flexor muscle.

Optionally, the motion activity of the wearer comprises finger movements.

Optionally, an inertial measurement unit (IMU) of the wrist-worn device reports less motion activity than that associated with the EMG signal obtained.

Optionally, the EMG electrode is disposed proximate to an artery of the wearer of the wrist-worn device, and wherein the artery is one of an ulnar artery or a radial artery.

Optionally, more than two EMG electrodes are disposed in or on the band of the wrist-worn device.

Optionally, the method further comprises selecting at least one EMG electrode of the more than two EMG electrodes from which to obtain an EMG for use in generating the modified PPG signal.

According to a further aspect of the present invention there is provided a method of determining physiological characteristics, the method comprising: obtaining an electromyography (EMG) signal representing muscle activity from an electromyography (EMG) electrode disposed in or on a band of a wrist-worn device, and a photoplethysmography (PPG) signal using a PPG sensor of the wrist-worn device; characterizing motion activity of a wearer of the wrist-worn device based on the EMG signal; and reporting a physiological characteristic determined using the PPG signal based at least in part on the characterization of the motion activity of the wearer.

Optionally, the physiological characteristic is at least one of: a heart rate of the wearer, a blood pressure of the wearer, or an oxygen saturation of the wearer.

Optionally, the method further comprises determining a manner in which the physiological characteristic is to be reported based on a type of physiological characteristic.

Optionally, reporting of the physiological characteristic comprises at least one of: presenting an indication of the physiological characteristic with a warning, or inhibiting presentation of an indication of the physiological characteristic.

Optionally, characterizing the motion activity comprises determining a type of motion activity.

Optionally, the method further comprisies comparing the PPG signal to an expected PPG signal selected based on the type of motion activity to form a comparison, wherein the reporting of the physiological characteristic is based on the comparison of the PPG signal to the expected PPG signal.

According to a further aspect of the present invention there is provided a wrist-worn device, the wrist-worn device comprising: at least one electromyography (EMG) electrode configured to generate EMG signals; at least one photoplethysmography (PPG) sensor configured to generate PPG signals; and at least one controller configured to: obtain an EMG signal representing muscle activity from the at least one EMG electrode disposed in or on a band of the wrist-worn device, and a PPG signal using the at least one PPG sensor of the wrist-worn device, generate a modified PPG signal using the EMG signal, wherein the modified PPG signal corrects for motion artifacts in the PPG signal due to motion activity of a wearer of the wrist-worn device, and determine at least one physiological characteristic based on the modified PPG signal.

Optionally, the at least one physiological characteristic is at least one of: a heart rate of the wearer, a blood pressure of the wearer, or an oxygen saturation of the wearer.

Optionally, the muscle activity comprises activity from at least one wrist extensor muscle or at least one wrist flexor muscle.

Optionally, the motion activity of the wearer comprises finger movements.

Optionally, an inertial measurement unit (IMU) of the wrist-worn device reports less motion activity than that associated with the EMG signal obtained.

Optionally, the EMG electrode is disposed proximate to an artery of the wearer of the wrist-worn device, and wherein the artery is one of an ulnar artery or a radial artery.

Disclosed herein are methods, systems, and media for determining physiological characteristics. In some embodiments, a method for determining physiological characteristics may involve obtaining an electromyography (EMG) signal representing muscle activity from an EMG electrode disposed in or on a band of a wrist-worn device, and a photoplethysmography (PPG) signal using a PPG sensor of the wrist-worn device. The method may further involve generating a modified PPG signal using the EMG signal, wherein the modified PPG signal corrects for motion artifacts in the PPG signal due to motion activity of a wearer of the wrist-worn device. The method may further involve determining at least one physiological characteristic based on the modified PPG signal.

In some embodiments, a method for determining physiological characteristics may involve obtaining an electromyography (EMG) signal representing muscle activity from an electromyography (EMG) electrode disposed in or on a band of a wrist-worn device, and a photoplethysmography (PPG) signal using a PPG sensor of the wrist-worn device. The method may further involve characterizing motion activity of a wearer of the wrist-worn device based on the EMG signal. The method may further involve reporting a physiological characteristic determined using the PPG signal based at least in part on the characterization of the motion activity of the wearer.

In some embodiments, a wrist-worn device may comprise at least one electromyography (EMG) electrode configured to generate EMG signals; at least one photoplethysmography (PPG) sensor configured to generate PPG signals; and at least one controller. The at least one controller may be configured to: obtain an EMG signal representing muscle activity from the at least one EMG electrode disposed in or on a band of the wrist-worn device, and a PPG signal using the at least one PPG sensor of the wrist-worn device; generate a modified PPG signal using the EMG signal, wherein the modified PPG signal corrects for motion artifacts in the PPG signal due to motion activity of a wearer of the wrist-worn device; and determine at least one physiological characteristic based on the modified PPG signal.

### BRIEF DESCRIPTION OF THE DRAWINGS

Illustrative embodiments are described in detail below with reference to the following figures.
FIG. 1A is a plan view of an example wristband system in accordance with some embodiments.
FIG. 1B is a schematic diagram of an example wrist-worn device in accordance with some embodiments.
FIG. 2 is a flowchart of an example process for utilizing electromyography (EMG) signals for motion artifact correction when determining a physiological characteristic in accordance with some embodiments.
FIG. 3 is a flowchart of an example process for utilizing EMG signals for gating recording of physiological characteristics in accordance with some embodiments.
FIG. 4 is a simplified block diagram of an example of a computing system that may be implemented as part of a user device according to certain embodiments.
FIG. 5 is a simplified block diagram of an example of a computing system that may be implemented as part of a server according to certain embodiments.

The figures depict embodiments of the present disclosure for purposes of illustration only. One skilled in the art will readily recognize from the following description that alternative embodiments of the structures and methods illustrated may be employed without departing from the principles, or benefits touted, of this disclosure.

In the appended figures, similar components and/or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.

### DETAILED DESCRIPTION

Wearable devices may be configured to determine physiological characteristics of a wearer of the device, such as a heart rate, respiration rate, heart rate variability, blood pressure, oxygen saturation, etc. Examples of such wearable devices include smart watches, fitness trackers, wrist-bands, etc. Because these physiological characteristics are intricately related to health of the user, accuracy is important. For example, reporting an inaccurate physiological characteristic, such as an inaccurate oxygen saturation or an inaccurate blood pressure, may cause the user to either become unnecessarily distressed (by inaccurately reporting an abnormal value of the physiological characteristic) or cause the user to ignore a medical problem (by inaccurately reporting a normal value of the physiological characteristic). Accordingly, accuracy in measurement, as well as appropriately reporting physiological measurements, is important.

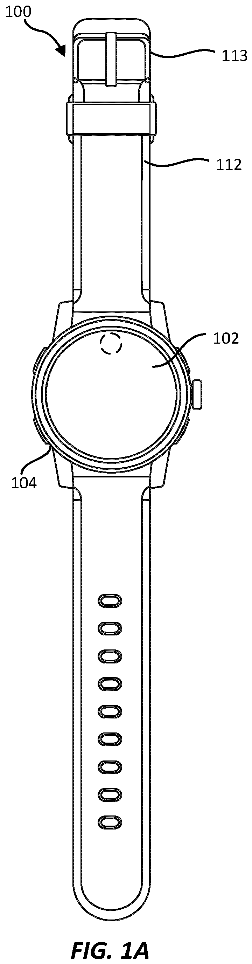

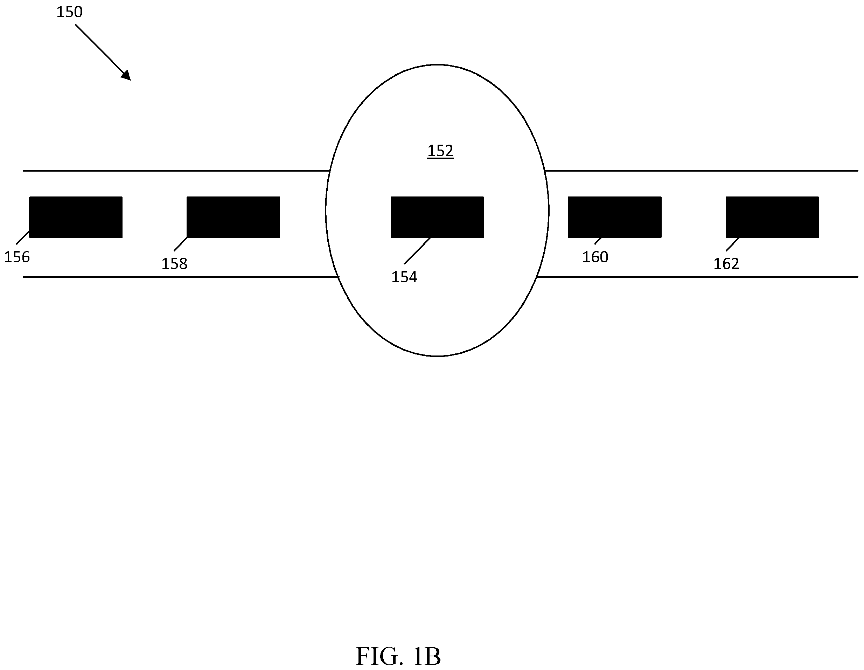

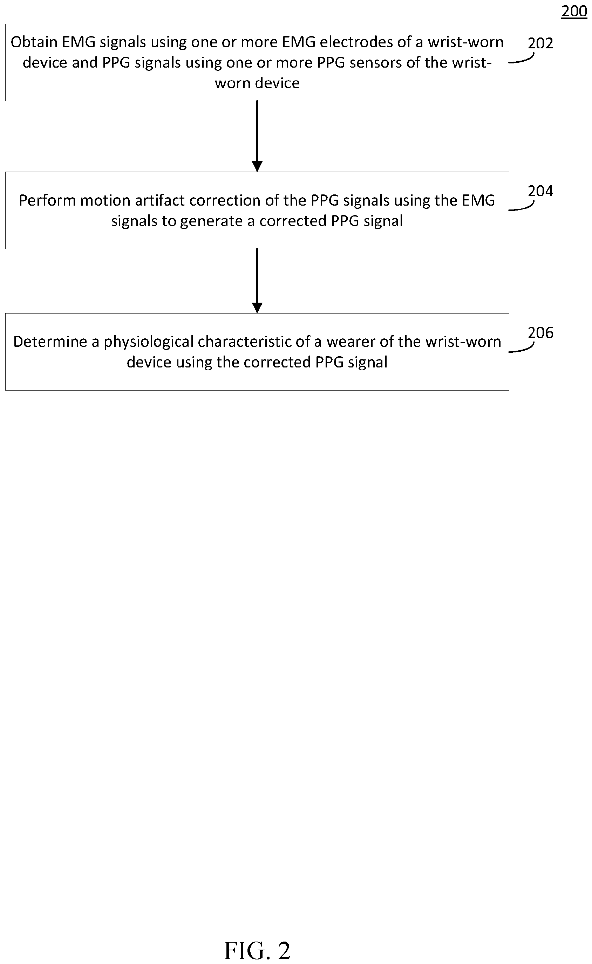

Disclosed herein are techniques for utilizing electromyography (EMG) signals in conjunction with physiological measurements. In particular, as shown in and described below in connection with FIGS. 1A and 1B, the physiological measurements may be made using at least photoplethysmography (PPG) signals. The PPG signals may be obtained using one or more PPG sensors disposed in or on a wrist-worn device (e.g., a smart watch, a fitness tracker, etc.), as shown in and described below in connection with FIGS. 1A and 1B. The wrist-worn device may additionally have one or more EMG electrodes configured to obtain EMG signals. These EMG signals may be used to perform motion artifact correction on the obtained PPG signals, which in turn allows more accurate determination of the physiological characteristics using the corrected PPG signals, as shown in and described below in connection with FIG. 2. Additionally or alternatively, in some implementations, the EMG signals may be used to modify reporting of physiological characteristics determined using PPG signals. For example, reporting of the physiological characteristics may be modified based on a likelihood of accuracy of the physiological measurement, where the likelihood of accuracy is dependent on an amount and/or a type of motion determined using the EMG signals.

The techniques described herein may be particularly useful for improving accuracy of PPG-based physiological measurements and/or controlling reporting of physiological measurements based on likely accuracy in instances in which the wearer of a wrist-worn device is engaging in motion activity that is not captured by an inertial measurement unit (IMU) of the wrist-worn device. In particular, while an IMU may capture movements that entail arm movements, the IMU may not detect or capture data related to finger movement or movement associated with small muscles in the wrist (e.g., wrist extensor or wrist flexor movements), such as typing, writing, knitting or sewing, etc. However, these relative smaller movements may still have a large effect on PPG-based physiological measurements, particularly when the PPG signals are obtained from a wrist-worn device. Accordingly, the techniques described herein may be particularly useful in situations in which a user is engaged in activities such as typing, writing, knitting, sewing, holding an object, etc.

It should be understood that the techniques described herein utilize one or more EMG electrodes disposed in or on a wrist-worn device. The one or more EMG electrodes may be used to obtain EMG signals that are utilized in various ways other than to improve accuracy of PPG-based physiological measurements and/or to modify reporting of PPG-based physiological measurements based on likely accuracy. For example, the EMG signals may be used for gesture control, e.g., in conjunction with use of a paired or associated virtual reality (VR) or augmented reality (AR) headset. In other words, the EMG electrodes used to implement the techniques herein may be used for various other purposes and by various other devices other than the wrist-worn device on which the EMG electrodes are disposed.

As described above, in some implementations, the wrist-worn device may be a smart watch. FIG. 1A illustrates an example wristband system 100 that includes a watch body 104 coupled to a watch band 112. Watch body 104 and watch band 112 may have any size and/or shape that is configured to allow a user to wear wristband system 100 on a body part (e.g., a wrist). Wristband system 100 may include a retaining mechanism 113 (e.g., a buckle) for securing watch band 112 to the user's wrist. Information, such as the time, date, measured user characteristics (e.g., physiological characteristics), etc. may be displayed on display 102. Display 102 may be a touchscreen such that the user may navigate through, e.g., menus, by touching portions of display 102. Wristband system 100 may perform various functions associated with the user. The functions may be executed independently in watch body 104, independently in watch band 112, and/or in communication between watch body 104 and watch band 112. Watch band 112 may be configured to operate independently (e.g., execute functions independently) from watch body 104. Additionally or alternatively, watch body 104 may be configured to operate independently (e.g., execute functions independently) from watch band 112. In some implementations, watch band 112 and/or watch body 104 may each include the independent resources required to independently execute functions. For example, watch band 112 and/or watch body 104 may each include a power source (e.g., a battery), a memory, data storage, a processor (e.g., a CPU), communications, a light source (e.g., at least one infrared LED for tracking watch body 104 and/or watch band 112 in space with an external sensor), and/or input/output devices.

FIG. 1B illustrates an example wrist-worn device 150 with multiple EMG electrodes. Note that FIG. 1B illustrates a backside portion of wrist-worn device 150 that is configured to be in contact with the skin of the wearer's wrist. As illustrated, wrist-worn device 150 includes a capsule 152. Capsule 152 includes a sensor 154, which may be a PPG sensor and/or an EMG electrode. Multiple sensors and/or electrodes may be disposed in or on capsule 152. The band of the wrist-worn device may include multiple EMG electrodes 156, 158, 160, and/or 162. Note that although the EMG electrodes are depicted as being linearly arranged along the band, in some implementations, the EMG electrodes may be in pairs along the band, and/or in any other suitable arrangement.

Wrist-worn devices (e.g., smart watches, fitness trackers, etc.) may be configured to determine various physiological characteristics of a wearer of the wrist-worn device. Examples of physiological characteristics include: heart rate, blood pressure (e.g., systolic and/or diastolic blood pressures), oxygen saturation, respiration rate, heart rate variability, neuromuscular activity, and the like. In many cases, some physiological characteristics may be determined using PPG signals. PPG signals may be determined by emitting light using one or more light emitters toward tissue or skin of a wearer and determining characteristics of the reflected light using one or more light detectors. The PPG signal itself may generally indicate changes in blood volume, and, based on these changes in blood volume, heart rate, blood pressure, and/or oxygen saturation may be determined. However, the PPG signal may become corrupted due to motion artifacts, which can in turn lead to inaccuracies in physiological characteristics (e.g., heart rate, blood pressure, oxygen saturation, etc.) determined used the corrupted PPG signal. As one example, a heart rate measured during walking or running may be inaccurate due to corruption of the PPG signal from motion artifacts. In particular, if a user is moving at, e.g., 2 Hz (120 steps per minute), a PPG signal that indicates a heart rate of 1 Hz (60 beats per minute) may be corrupted by a 2 Hz signal from the motion activity. Continuing with this example, because the PPG signal may contain a mix of 1 Hz (corresponding to the true heart rate of 60 beats per minute) and 2 Hz (corresponding to the motion activity) signals, a heart rate determined based on this corrupted signal may be inaccurate, and may be determined as, e.g., a heart rate between 60 - 120 beats per minute, as a heart rate of 120 beats per minute, or the like.

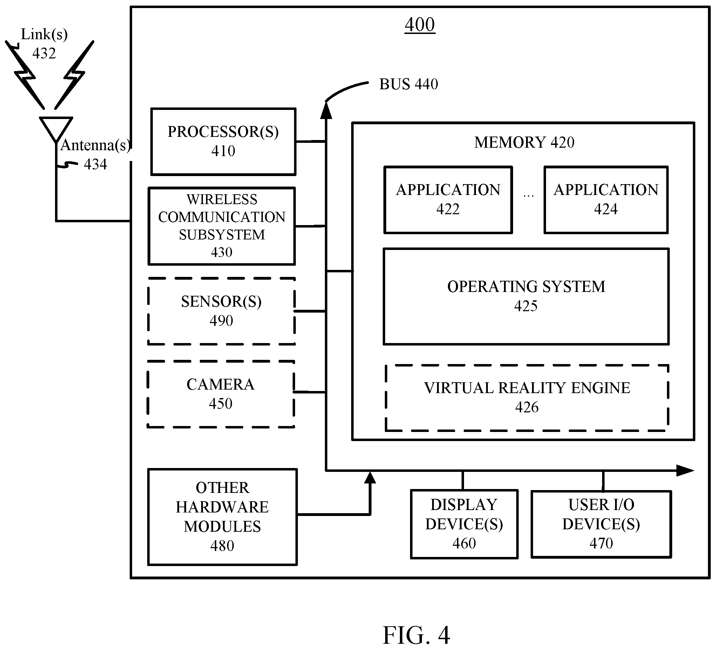

Conventional techniques may utilize motion signals from an inertial measurement unit (IMU) to correct PPG signals to account for motion artifacts. As described below in connection with FIG. 4, the IMU may include various motion sensors, such as an accelerometer, a gyroscope, a magnetometer, etc. The PPG signal may then be corrected based on data acquired by the IMU. Using the example given above of walking or running activity having a 2 Hz signal and a true heart rate corresponding to 1 Hz (e.g., 60 beats per minute), the IMU activity may be used to determine that the motion activity corresponds to a 2 Hz signal. Continuing with this example, a frequency domain representation of the IMU activity (which indicates the 2 Hz signal) may be subtracted from a frequency domain representation of the PPG signal (which includes a mix of the 1 Hz heart rate signal and the 2 Hz motion activity), leaving the 1 Hz heart rate signal in the modified (or corrected) PPG signal. The heart rate may then be determined based on the corrected PPG signal, which has effectively had the motion activity subtracted out or removed.

However, in cases in which there is motion activity during acquisition of the PPG signal but in which the IMU does not accurately capture the motion activity, utilizing IMU data to correct the PPG signal may not adequately correct the PPG signal for motion artifacts. For example, in an instance in which the IMU is disposed in or on a wrist-worn device, and in which the motion activity primarily involves finger movements or wrist movements (e.g., typing, writing, holding an object, etc.), the PPG signal obtained from one or more PPG sensors of the wrist-worn device may be corrupted by the motion activity while not captured by the IMU data. Accordingly, correcting the PPG signal using IMU data may not adequately correct for motion artifacts, because the motion activity is not represented in the IMU data.

Disclosed herein are techniques for correcting PPG signals to account for motion artifacts using EMG signals. The EMG signals may indicate neuromuscular activity. For example, for EMG electrodes disposed in or on a wrist-worn device (e.g., in or on the band, in or on the back capsule, etc.) as shown in and described above in connection with FIG. 1B, the EMG signals may indicate neuromuscular activity of wrist flexor or wrist extensor muscles, of muscles that control finger movement, etc. Accordingly, the EMG signals may capture motion activity that is not captured by the IMU for activities such as typing, writing, playing an instrument, or the like. As described herein, the EMG signals may be used to correct a PPG signal. The corrected PPG signal may then be used to determine various physiological characteristics, such as heart rate, blood pressure, oxygen saturation, or the like. It should be understood that, as used herein, "correcting" for motion artifacts may not completely remove all motion artifacts. In other words, a corrected PPG signal may still include some motion artifact that is less than the motion artifact present in the originally obtained PPG signal (e.g., the motion artifact correction may be a partial motion artifact correction).

**FIG. 2** is a flowchart of an example process 200 for performing motion artifact correction of PPG signals based on EMG signals in accordance with some embodiments. Blocks of process 200 may be performed by a processor and/or a controller associated with a wrist-worn device. In some implementations, blocks of process 200 may be performed in an order other than what is shown in FIG. 2. In some embodiments, two or more blocks of process 200 may be performed substantially in parallel. In some embodiments, one or more blocks of process 200 may be omitted.

Process 200 can begin at 202 by obtaining EMG signals using one or more EMG electrodes of a wrist-worn device and PPG signals using one or more PPG sensors of the wrist-worn device. The one or more EMG electrodes may be disposed in or on the band and/or the back portion of the capsule of the wrist-worn device, as shown in and described above in connection with FIG. 1B. The one or more EMG electrodes may be configured to obtain EMG signals representing neuromuscular signals associated with wrist flexor and/or wrist extensor muscles. The one or more PPG sensors may include one or more light emitters and/or one or more light detectors. Light may be emitted at any suitable wavelength, e.g., in the green wavelength, in the red wavelength, in the infrared wavelength, etc. In some implementations, light may be emitted at multiple wavelengths. The one or more PPG sensors may be disposed in or on a back portion of the capsule of the wrist-worn device configured to be proximate to or in contact with the wrist of the wearer. In some embodiments, the EMG signals and the PPG signals may be obtained in a synchronized manner such that time points of the EMG signals can be aligned with time points of the PPG signals. For example, the EMG signals and the PPG signals may be obtained using synchronized clocks. As another example, the EMG signals and the PPG signals may be received by and/or processed by the same analog front end (AFE) circuitry.

It should be noted that, in some implementations, acquisition of the EMG signals and/or the PPG signals may be automatic, e.g., without user request or input. Additionally or alternatively, in some implementations, acquisition of the EMG signals and/or the PPG signals may be responsive to a specific user request, e.g., to determine a particular physiological characteristic of the user. Such a request may be obtained via input control buttons or input control interfaces of the wrist-worn device.

At 204, process 200 can perform motion artifact correction of the PPG signals using the EMG signals to generate a corrected PPG signal. The motion artifact correction may be performed using various techniques or combinations of techniques. For example, in some implementations, the EMG signals and the PPG signals may be provided as input to a trained machine learning model that generates, as an output, the corrected PPG signal. In such implementations, the machine learning model may be trained offline using a training set of PPG signals that include motion artifacts, EMG signals, and ground truth PPG signals that correspond to the corrected PPG signals. By way of example, the PPG signals that include motion artifact and the EMG signals may be acquired from a first wrist of user where the fingers and/or wrist is in motion, and the ground truth PPG signal may be acquired using a wrist-worn device worn on the other wrist of the user.

As another example, in some implementations, the EMG signals may be provided to a trained machine learning model that generates, as an output, a signal to be subtracted from the acquired PPG signals to derive the corrected PPG signal. In such implementations, the machine learning model may be trained offline using a training set of EMG signals corresponding to motion artifacts and corresponding ground truth signals indicating a signal to be subtracted out of the acquired PPG signal.

Note that, in instances in which a trained machine learning model is used to generate the corrected PPG signal, the machine learning model may be trained offline on a remote device, such as a remote server. After training, parameters of the trained machine learning model (e.g., weights) may be transmitted to and stored on the wrist-worn device to be used at inference time. IN some implementations, a machine learning model may be trained using multiple users (e.g., two, five, ten, one hundred, etc.), where the multiple users may or may not include the wearer of the wrist-worn device of process 200.

At 206, process 200 can determine a physiological characteristic of a wearer of the wrist-worn device using the corrected PPG signal. As described above, the physiological characteristic may include a heart rate, a heart rate variability, a blood pressure, an oxygen saturation, or the like. In some implementations, the physiological characteristic may be obtained by providing the corrected PPG signal to a trained machine learning model that generates, as an output, the physiological characteristic. In some implementations, the physiological characteristic may be obtained by performing a sequence of processing operations on the corrected PPG signal to extract, e.g., frequency content of the corrected PPG signal, power spectrum information of the corrected PPG signal, timing information associated with the corrected PPG signal, etc. The extracted information may then be used to determine the physiological characteristic (e.g., using a mathematical function or expression, a regression model, a look up table, or the like).

In some implementations, after determining the physiological characteristic, process 200 can cause an indication of the physiological characteristic to be presented to the wearer and/or stored. For example, the indication of the physiological characteristic may be presented on a face or display of the wrist-worn device. As another example, an indication of the physiological characteristic may be stored in memory of the wrist-worn device and/or transmitted to another device for stage (e.g., a paired user device such as a paired mobile phone, a cloud device, etc.).

In some implementations, physiological measurements obtained via one or more sensors of a wrist-worn device may be inaccurate, e.g., due to motion artifacts as described above. For certain types of physiological measurements, reporting inaccurate physiological measurements may be harmful. For example, an inaccurate blood pressure measurement may cause undue distress (e.g., if the measurement is inaccurately measured as substantially higher than the actual blood pressure) or may conversely inhibit the wearer from seeking necessary medical treatment (e.g., if the measurement is inaccurately measured as substantially lower than the actual blood pressure). Similar problems may occur with inaccurate oxygen saturation measurements.

As described above, motion activity is conventionally detected using an IMU of the device. However, IMU data may not accurately detect certain types of motion activity, such as typing, writing, playing an instrument, etc., because the motion may primarily occur in the fingers or small wrist muscles, and is therefore not detectable via the motion sensors of the IMU disposed in the wrist-worn device. However, even if the motion activity is not detected by the IMU, PPG signals may still be corrupted due to motion artifact, as described above.

Disclosed herein are techniques for gating reporting of physiological characteristics based on characterization of motion activity detected via EMG signals. In some implementations, PPG signals may be obtained using one or more PPG sensors disposed in or on the wrist-band of the device. The PPG signals may be used to determine various physiological characteristics, such as heart rate, heart rate variability, blood pressure, oxygen saturation, etc. Concurrently, EMG signals may be obtained via one or more EMG electrodes disposed in or on the wrist-worn device as shown in and described above in connection with FIG. 1B. In some implementations, the EMG signals may be utilized to characterize motion activity associated with a wearer of the wrist-worn device. For example, characterizing the motion activity may involve determining a type of motion activity (e.g., typing, writing, exercising, playing an instrument, knitting or sewing, etc.). Based on the characterization of the motion activity, reporting of the physiological measurement may be modified. For example, the physiological measurement may be reported with an alert or warning indicating that the physiological measurement may not be accurate due to motion activity. As another example, reporting of the physiological measurement may be inhibited completely. In some implementations, a manner in which reporting of the physiological measurement is modified may be responsive to the type of motion activity detected using the EMG signals and/or an amount of motion activity detected using the EMG signals. Additionally or alternatively, in some implementations, determination of whether and how to modify reporting of the physiological measurements may be determined based at least in part on comparing the acquired PPG signals to expected PPG signals to quantify the degradation in the acquired PPG signals.

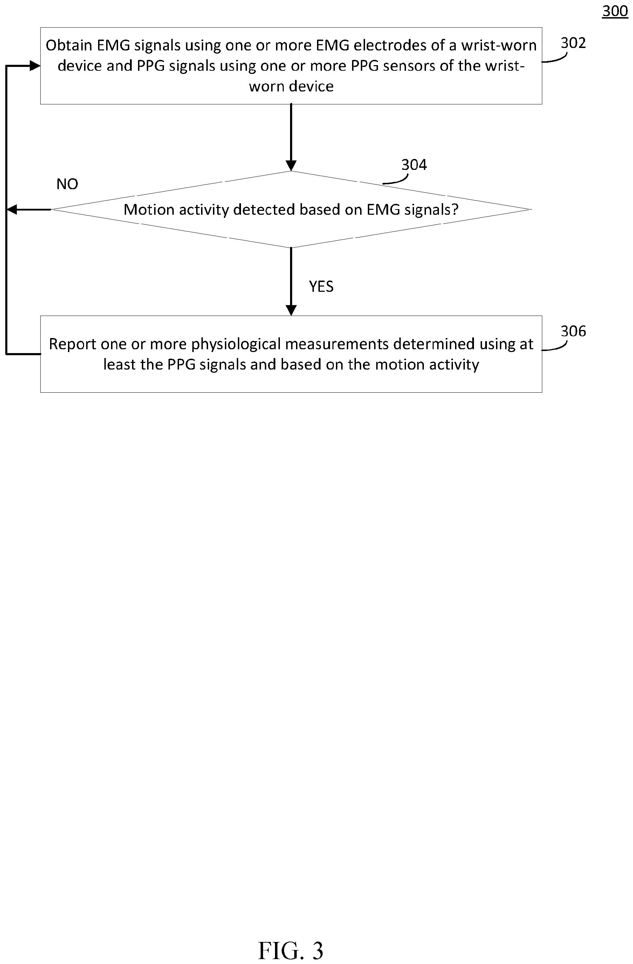

**FIG. 3** is a flowchart of an example process 300 for modifying reporting of physiological measurements based on EMG signals in accordance with some embodiments. In some implementations, blocks of process 300 may be executed by a processor and/or a controller of a wrist-worn device. In some embodiments, blocks of process 300 may be performed in an order other than what is shown in FIG. 3. In some embodiments, two or more blocks of process 300 may be performed substantially in parallel. In some embodiments, one or more blocks of process 300 may be omitted.

Process 300 can begin at 302 by obtaining EMG signals using one or more EMG electrodes of a wrist-worn device and PPG signals using one or more PPG sensors of the wrist-worn device. The one or more EMG electrodes may be disposed in or on the band and/or the back portion of the capsule of the wrist-worn device, as shown in and described above in connection with FIG. 1B. The one or more EMG electrodes may be configured to obtain EMG signals representing neuromuscular signals associated with wrist flexor and/or wrist extensor muscles. The one or more PPG sensors may include one or more light emitters and/or one or more light detectors. Light may be emitted at any suitable wavelength, e.g., in the green wavelength, in the red wavelength, in the infrared wavelength, etc. In some implementations, light may be emitted at multiple wavelengths. The one or more PPG sensors may be disposed in or on a back portion of the capsule of the wrist-worn device configured to be proximate to or in contact with the wrist of the wearer. In some embodiments, the EMG signals and the PPG signals may be obtained in a synchronized manner such that time points of the EMG signals can be aligned with time points of the PPG signals. For example, the EMG signals and the PPG signals may be obtained using synchronized clocks. As another example, the EMG signals and the PPG signals may be received by and/or processed by the same analog front end (AFE) circuitry.

It should be noted that, in some implementations, acquisition of the EMG signals and/or the PPG signals may be automatic, e.g., without user request or input. Additionally or alternatively, in some implementations, acquisition of the EMG signals and/or the PPG signals may be responsive to a specific user request, e.g., to determine a particular physiological characteristic of the user. Such a request may be obtained via input control buttons or input control interfaces of the wrist-worn device.

At 304, process 300 can determine whether any motion activity is detected based on the EMG signals. In some implementations, process 300 can determine whether any motion activity is detected based on the magnitude of the EMG signals. For example, the magnitude may be an average magnitude, a maximum amplitude, or the like over a given time window. In some implementations, motion activity may be detected responsive to a metric (e.g., average magnitude, maximum amplitude, etc.) of the EMG signals exceeding a predetermined threshold.

If, at 304, process 300 determines that motion activity has not been detected ("no" at 304), process 300 can loop back to 302 and can obtain additional EMG signals and additional PPG signals.

Conversely, if, at 304, process 300 determines that motion activity has been detected ("yes" at 304), process 300 can proceed to 306 and can report one or more physiological measurements determined using at least the PPG signals based on the motion activity. Reporting of one or more physiological measurements may involve presenting an indication of the physiological measurement with a warning or alert indicating that the measurement may be inaccurate, inhibiting reporting of the physiological measurement altogether, or the like. In some implementations, a manner in which reporting of the one or more physiological measurements occurs may be determined based on: a type of motion activity detected using the EMG signals; the type of physiological measurement; a magnitude of the motion activity; a difference between the obtained PPG signals and expected PPG signals (e.g., expected in a no motion condition, expected for the given detected motion activity, or the like); or any combination thereof.

For example, in some implementations, a manner in which physiological measurements are reported may be selected based on the type of motion activity. Example types of motion activity include typing, writing, holding an object, knitting/sewing, exercise, or the like. In some embodiments, physiological measurements may be reported in one manner for certain types of motion activity (e.g., typing or writing) and in a different manner for other types of motion activity (e.g., holding an object). In some implementations, the type of motion activity may be determined by providing the EMG signals to a trained machine learning model that classifies the motion activity as associated with a particular class or type of motion activity. Such a machine learning model may be trained on a remote device (e.g., a server), and, once trained, parameters (e.g., weights) associated with the model may be transmitted to and stored on the wrist-worn device for use during inference time.

As another example, in some implementations, a manner in which physiological measurements are reported may be selected based on the type of physiological measurement. Example types of physiological measurement include heart rate measurement, heart rate variability measurement, respiration rate measurement, blood pressure measurement, oxygen saturation measurement, etc. By way of example, in some embodiments, reporting of certain types of physiological measurement (e.g., heart rate, heart rate variability, etc.) may be modified in one manner (e.g., reported with a warning that the measurement may be accurate), whereas reporting of other types of physiological measurement (e.g., blood pressure, oxygen saturation, etc.) may be modified in a different manner (e.g., inhibiting reporting of the measurement altogether).

As yet another example, in some implementations, a manner in which physiological measurements are reported may be selected based on the magnitude of the motion activity. For example, the physiological measurement may be reported with a warning indicating possible inaccuracy responsive to the magnitude of the motion activity being below a predetermined threshold. Conversely, responsive to the magnitude of the motion activity being above a predetermined threshold, the physiological measurement may be inhibited from being reported at all.

At still another example, in some implementations, a manner in which physiological measurements are reported may be selected based on a difference between the acquired PPG signals and expected PPG signals. The expected PPG signals may be the expected PPG signals under a condition of no or little motion, or the expected PPG signals for the given amount of motion detected using the EMG signals. By way of example, in an instance in which the difference between the acquired PPG signals and the expected PPG signals is greater than a threshold amount, process 300 may inhibit reporting of the physiological measurement. Conversely, in an instance in which the difference between the acquired PPG signals and the expected PPG signals is less than the threshold amount, process 300 may report the physiological measurement with a warning.

Process 300 can then loop back to block 302 and can obtain additional EMG signals and additional PPG signals.

**FIG. 4** is a simplified block diagram of an example of a computing system 400 for implementing some of the examples described herein. For example, in some embodiments, computing system may be used to implement a user device (e.g., a mobile phone, a tablet computer, a wrist-worn device, etc.) that implements the blocks of processes 200 and 300 of FIGS. 2 and 3, respectively. In the illustrated example, computing system 400 may include one or more processor(s) 410 and a memory 420. Processor(s) 410 may be configured to execute instructions for performing operations at a number of components, and can be, for example, a general-purpose processor or microprocessor suitable for implementation within a portable electronic device. Processor(s) 410 may be communicatively coupled with a plurality of components within computing system 400. To realize this communicative coupling, processor(s) 410 may communicate with the other illustrated components across a bus 440. Bus 440 may be any subsystem adapted to transfer data within computing system 400. Bus 440 may include a plurality of computer buses and additional circuitry to transfer data.

Memory 420 may be coupled to processor(s) 410. In some embodiments, memory 420 may offer both short-term and long-term storage and may be divided into several units. Memory 420 may be volatile, such as static random access memory (SRAM) and/or dynamic random access memory (DRAM) and/or non-volatile, such as read-only memory (ROM), flash memory, and the like. Furthermore, memory 420 may include removable storage devices, such as secure digital (SD) cards. Memory 420 may provide storage of computer-readable instructions, data structures, program modules, and other data for computing system 400. In some embodiments, memory 420 may be distributed into different hardware modules. A set of instructions and/or code might be stored on memory 420. The instructions might take the form of executable code that may be executable by computing system 400, and/or might take the form of source and/or installable code, which, upon compilation and/or installation on computing system 400 (e.g., using any of a variety of generally available compilers, installation programs, compression/decompression utilities, etc.), may take the form of executable code.

In some embodiments, memory 420 may store a plurality of application modules 422 through 424, which may include any number of applications. Examples of applications may include gaming applications, conferencing applications, video playback applications, or other suitable applications. The applications may include a depth sensing function or eye tracking function. Application modules 422-424 may include particular instructions to be executed by processor(s) 410. In some embodiments, certain applications or parts of application modules 422-424 may be executable by other hardware modules 480. In certain embodiments, memory 420 may additionally include secure memory, which may include additional security controls to prevent copying or other unauthorized access to secure information.

In some embodiments, memory 420 may include an operating system 425 loaded therein. Operating system 425 may be operable to initiate the execution of the instructions provided by application modules 422-424 and/or manage other hardware modules 480 as well as interfaces with a wireless communication subsystem 430 which may include one or more wireless transceivers. Operating system 425 may be adapted to perform other operations across the components of computing system 400 including threading, resource management, data storage control and other similar functionality.

Wireless communication subsystem 430 may include, for example, an infrared communication device, a wireless communication device and/or chipset (such as a Bluetooth° device, an IEEE 802.11 device, a Wi-Fi device, a WiMax device, cellular communication facilities, etc.), and/or similar communication interfaces. Computing system 400 may include one or more antennas 434 for wireless communication as part of wireless communication subsystem 430 or as a separate component coupled to any portion of the system. Depending on desired functionality, wireless communication subsystem 430 may include separate transceivers to communicate with base transceiver stations and other wireless devices and access points, which may include communicating with different data networks and/or network types, such as wireless wide-area networks (WWANs), wireless local area networks (WLANs), or wireless personal area networks (WPANs). A WWAN may be, for example, a WiMax (IEEE 802.17) network. A WLAN may be, for example, an IEEE 802.11x network. A WPAN may be, for example, a Bluetooth network, an IEEE 802.7x, or some other types of network. The techniques described herein may also be used for any combination of WWAN, WLAN, and/or WPAN. Wireless communications subsystem 430 may permit data to be exchanged with a network, other computer systems, and/or any other devices described herein. Wireless communication subsystem 430 may include a means for transmitting or receiving data, such as identifiers of HMD devices, position data, a geographic map, a heat map, photos, or videos, using antenna(s) 434 and wireless link(s) 432. Wireless communication subsystem 430, processor(s) 410, and memory 420 may together comprise at least a part of one or more of a means for performing some functions disclosed herein.

Embodiments of computing system 400 may also include one or more sensors 490. Sensor(s) 490 may include, for example, an image sensor, an accelerometer, a force sensor, a hydrostatic pressure sensor, a temperature sensor, a proximity sensor, a magnetometer, a gyroscope, an inertial sensor (e.g., a module that combines an accelerometer and a gyroscope), an ambient light sensor, or any other similar module operable to provide sensory output and/or receive sensory input, such as a depth sensor or a position sensor. For example, in some implementations, sensor(s) 490 may include one or more inertial measurement units (IMUs) and/or one or more position sensors. An IMU may generate calibration data indicating an estimated position of a device, based on measurement signals received from one or more of the position sensors. A position sensor may generate one or more measurement signals in response to motion of a device. Examples of the position sensors may include, but are not limited to, one or more accelerometers, one or more gyroscopes, one or more magnetometers, another suitable type of sensor that detects motion, a type of sensor used for error correction of the IMU, or some combination thereof. The position sensors may be located external to the IMU, internal to the IMU, or some combination thereof. At least some sensors may use a structured light pattern for sensing.

Computing system 400 may include a display module 460. Display module 460 may be a near-eye display, and may graphically present information, such as images, videos, and various instructions, from computing system 400 to a user. Such information may be derived from one or more application modules 422-424, virtual reality engine 426, one or more other hardware modules 480, a combination thereof, or any other suitable means for resolving graphical content for the user (e.g., by operating system 425). Display module 460 may use liquid crystal display (LCD) technology, light-emitting diode (LED) technology (including, for example, OLED, ILED, mLED, AMOLED, TOLED, etc.), light emitting polymer display (LPD) technology, or some other display technology.

Computing system 400 may include a user input/output module 470. User input/output module 470 may allow a user to send action requests to computing system 400. An action request may be a request to perform a particular action. For example, an action request may be to start or end an application or to perform a particular action within the application. User input/output module 470 may include one or more input devices. Example input devices may include a touchscreen, a touch pad, microphone(s), button(s), dial(s), switch(es), a keyboard, a mouse, a game controller, or any other suitable device for receiving action requests and communicating the received action requests to computing system 400. In some embodiments, user input/output module 470 may provide haptic feedback to the user in accordance with instructions received from computing system 400. For example, the haptic feedback may be provided when an action request is received or has been performed.

Computing system 400 may include a camera 450 that may be used to take photos or videos. Camera 450 may be configured to take photos or videos of the user. Camera 450 may also be used to take photos or videos of the environment, for example, for VR, AR, or MR applications. Camera 450 may include, for example, a complementary metal-oxide-semiconductor (CMOS) image sensor with a few millions or tens of millions of pixels. In some implementations, camera 450 may include two or more cameras that may be used to capture 3-D images.

In some embodiments, computing system 400 may include a plurality of other hardware modules 480. Each of other hardware modules 480 may be a physical module within computing system 400. While each of other hardware modules 480 may be permanently configured as a structure, some of other hardware modules 480 may be temporarily configured to perform specific functions or temporarily activated. Examples of other hardware modules 480 may include, for example, an audio output and/or input module (e.g., a microphone or speaker), a near field communication (NFC) module, a rechargeable battery, a battery management system, a wired/wireless battery charging system, etc. In some embodiments, one or more functions of other hardware modules 480 may be implemented in software.

In some embodiments, memory 420 of computing system 400 may also store a virtual reality engine 426. Virtual reality engine 426 may execute applications within computing system 400 and receive position information, acceleration information, velocity information, predicted future positions, or some combination thereof from the various sensors. In some embodiments, the information received by virtual reality engine 426 may be used for producing a signal (e.g., display instructions) to display module 460. For example, if the received information indicates that the user has looked to the left, virtual reality engine 426 may generate content that mirrors the user's movement in a virtual environment. Additionally, virtual reality engine 426 may perform an action within an application in response to an action request received from user input/output module 470 and provide feedback to the user. The provided feedback may be visual, audible, or haptic feedback. In some implementations, processor(s) 410 may include one or more GPUs that may execute virtual reality engine 426.

In various implementations, the above-described hardware and modules may be implemented on a single device or on multiple devices that can communicate with one another using wired or wireless connections. For example, in some implementations, some components or modules, such as GPUs, virtual reality engine 426, and applications (e.g., tracking application), may be implemented on two or more paired or connected devices.

In alternative configurations, different and/or additional components may be included in computing system 400. Similarly, functionality of one or more of the components can be distributed among the components in a manner different from the manner described above. For example, in some embodiments, computing system 400 may be modified to include other system environments, such as an AR system environment and/or an MR environment.

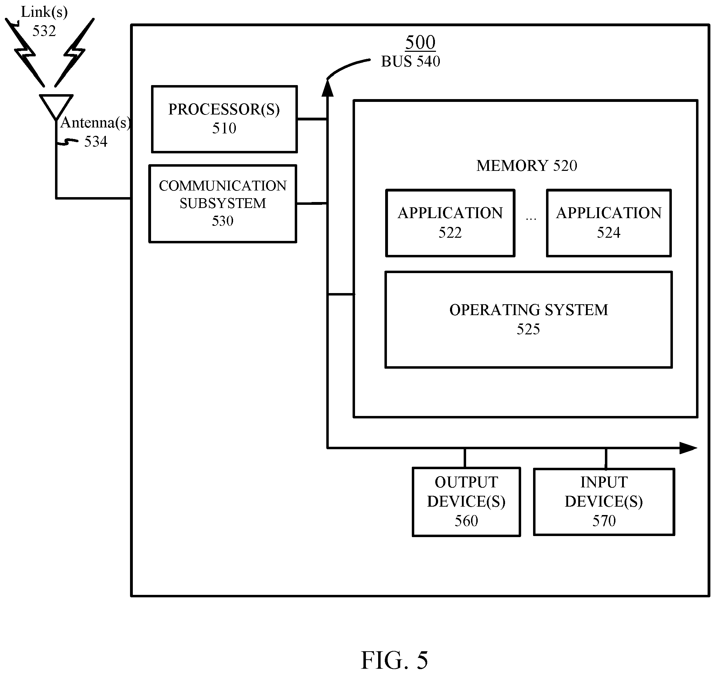

FIG. 5 is a simplified block diagram of an example of a computing system 500 that may be implemented in connection with a server in accordance with some embodiments. For example, computing system 500 may be used to implement a server used to train a model, e.g., that determines one or more physiological characteristics based on sensor measurements, or the like.

In the illustrated example, computing system 500 may include one or more processor(s) 510 and a memory 520. Processor(s) 510 may be configured to execute instructions for performing operations at a number of components, and can be, for example, a general-purpose processor or microprocessor suitable for implementation within a portable electronic device. Processor(s) 510 may be communicatively coupled with a plurality of components within computing system 500. To realize this communicative coupling, processor(s) 510 may communicate with the other illustrated components across a bus 540. Bus 540 may be any subsystem adapted to transfer data within computing system 500. Bus 540 may include a plurality of computer buses and additional circuitry to transfer data.

Memory 520 may be coupled to processor(s) 510. In some embodiments, memory 520 may offer both short-term and long-term storage and may be divided into several units. Memory 520 may be volatile, such as static random access memory (SRAM) and/or dynamic random access memory (DRAM) and/or non-volatile, such as read-only memory (ROM), flash memory, and the like. Furthermore, memory 520 may include removable storage devices, such as secure digital (SD) cards. Memory 520 may provide storage of computer-readable instructions, data structures, program modules, and other data for computing system 500. In some embodiments, memory 520 may be distributed into different hardware modules. A set of instructions and/or code might be stored on memory 520. The instructions might take the form of executable code that may be executable by computing system 500, and/or might take the form of source and/or installable code, which, upon compilation and/or installation on computing system 500 (e.g., using any of a variety of generally available compilers, installation programs, compression/decompression utilities, etc.), may take the form of executable code.

In some embodiments, memory 520 may store a plurality of application modules 522 through 524, which may include any number of applications. Examples of applications may include gaming applications, conferencing applications, video playback applications, or other suitable applications. Application modules 522- 524 may include particular instructions to be executed by processor(s) 510. In some embodiments, certain applications or parts of application modules 522- 524 may be executable by other hardware modules. In certain embodiments, memory 520 may additionally include secure memory, which may include additional security controls to prevent copying or other unauthorized access to secure information.

In some embodiments, memory 520 may include an operating system 525 loaded therein. Operating system 525 may be operable to initiate the execution of the instructions provided by application modules 522- 524 and/or manage other hardware modules as well as interfaces with a wireless communication subsystem 530 which may include one or more wireless transceivers. Operating system 525 may be adapted to perform other operations across the components of computing system 500 including threading, resource management, data storage control and other similar functionality.

Communication subsystem 530 may include, for example, an infrared communication device, a wireless communication device and/or chipset (such as a Bluetooth° device, an IEEE 802.11 device, a Wi-Fi device, a WiMax device, cellular communication facilities, etc.), a wired communication interface, and/or similar communication interfaces. Computing system 500 may include one or more antennas 534 for wireless communication as part of wireless communication subsystem 530 or as a separate component coupled to any portion of the system. Depending on desired functionality, communication subsystem 530 may include separate transceivers to communicate with base transceiver stations and other wireless devices and access points, which may include communicating with different data networks and/or network types, such as wireless wide-area networks (WWANs), wireless local area networks (WLANs), or wireless personal area networks (WPANs). A WWAN may be, for example, a WiMax (IEEE 802.17) network. A WLAN may be, for example, an IEEE 802.11x network. A WPAN may be, for example, a Bluetooth network, an IEEE 802.8x, or some other types of network. The techniques described herein may also be used for any combination of WWAN, WLAN, and/or WPAN. Communications subsystem 530 may permit data to be exchanged with a network, other computer systems, and/or any other devices described herein. Communication subsystem 530 may include a means for transmitting or receiving data, using antenna(s) 534, wireless link(s) 532, or a wired link. Communication subsystem 530, processor(s) 510, and memory 520 may together comprise at least a part of one or more of a means for performing some functions disclosed herein.

In some embodiments, computing system 500 may include one or more output device(s) 560 and/or one or more input device(s) 570. Output device(s) 570 and/or input device(s) 570 may be used to provide output information and/or receive input information.

Embodiments disclosed herein may be used to implement components of an artificial reality system or may be implemented in conjunction with an artificial reality system. Artificial reality is a form of reality that has been adjusted in some manner before presentation to a user, which may include, for example, a virtual reality, an augmented reality, a mixed reality, a hybrid reality, or some combination and/or derivatives thereof. Artificial reality content may include completely generated content or generated content combined with captured (e.g., real-world) content. The artificial reality content may include video, audio, haptic feedback, or some combination thereof, and any of which may be presented in a single channel or in multiple channels (such as stereo video that produces a three-dimensional effect to the viewer). Additionally, in some embodiments, artificial reality may also be associated with applications, products, accessories, services, or some combination thereof, that are used to, for example, create content in an artificial reality and/or are otherwise used in (e.g., perform activities in) an artificial reality. The artificial reality system that provides the artificial reality content may be implemented on various platforms, including an HMD connected to a host computer system, a standalone HMD, a mobile device or computing system, or any other hardware platform capable of providing artificial reality content to one or more viewers.

The methods, systems, and devices discussed above are examples. Various embodiments may omit, substitute, or add various procedures or components as appropriate. For instance, in alternative configurations, the methods described may be performed in an order different from that described, and/or various stages may be added, omitted, and/or combined. Also, features described with respect to certain embodiments may be combined in various other embodiments. Different aspects and elements of the embodiments may be combined in a similar manner. Also, technology evolves and, thus, many of the elements are examples that do not limit the scope of the disclosure to those specific examples.

Specific details are given in the description to provide a thorough understanding of the embodiments. However, embodiments may be practiced without these specific details. For example, well-known circuits, processes, systems, structures, and techniques have been shown without unnecessary detail in order to avoid obscuring the embodiments. This description provides example embodiments only, and is not intended to limit the scope, applicability, or configuration of the invention. Rather, the preceding description of the embodiments will provide those skilled in the art with an enabling description for implementing various embodiments. Various changes may be made in the function and arrangement of elements without departing from the scope of the present disclosure.

Also, some embodiments were described as processes depicted as flow diagrams or block diagrams. Although each may describe the operations as a sequential process, many of the operations may be performed in parallel or concurrently. In addition, the order of the operations may be rearranged. A process may have additional steps not included in the figure. Furthermore, embodiments of the methods may be implemented by hardware, software, firmware, middleware, microcode, hardware description languages, or any combination thereof. When implemented in software, firmware, middleware, or microcode, the program code or code segments to perform the associated tasks may be stored in a computer-readable medium such as a storage medium. Processors may perform the associated tasks.

It will be apparent to those skilled in the art that substantial variations may be made in accordance with specific requirements. For example, customized or special-purpose hardware might also be used, and/or particular elements might be implemented in hardware, software (including portable software, such as applets, etc.), or both. Further, connection to other computing devices such as network input/output devices may be employed.

With reference to the appended figures, components that can include memory can include non-transitory machine-readable media. The term "machine-readable medium" and "computer-readable medium" may refer to any storage medium that participates in providing data that causes a machine to operate in a specific fashion. In embodiments provided hereinabove, various machine-readable media might be involved in providing instructions/code to processing units and/or other device(s) for execution. Additionally or alternatively, the machine-readable media might be used to store and/or carry such instructions/code. In many implementations, a computer-readable medium is a physical and/or tangible storage medium. Such a medium may take many forms, including, but not limited to, non-volatile media, volatile media, and transmission media. Common forms of computer-readable media include, for example, magnetic and/or optical media such as compact disk (CD) or digital versatile disk (DVD), punch cards, paper tape, any other physical medium with patterns of holes, a RAM, a programmable read-only memory (PROM), an erasable programmable read-only memory (EPROM), a FLASH-EPROM, any other memory chip or cartridge, a carrier wave as described hereinafter, or any other medium from which a computer can read instructions and/or code. A computer program product may include code and/or machine-executable instructions that may represent a procedure, a function, a subprogram, a program, a routine, an application (App), a subroutine, a module, a software package, a class, or any combination of instructions, data structures, or program statements.

Those of skill in the art will appreciate that information and signals used to communicate the messages described herein may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, or any combination thereof.

Terms, "and" and "or" as used herein, may include a variety of meanings that are also expected to depend at least in part upon the context in which such terms are used. Typically, "or" if used to associate a list, such as A, B, or C, is intended to mean A, B, and C, here used in the inclusive sense, as well as A, B, or C, here used in the exclusive sense. In addition, the term "one or more" as used herein may be used to describe any feature, structure, or characteristic in the singular or may be used to describe some combination of features, structures, or characteristics. However, it should be noted that this is merely an illustrative example and claimed subject matter is not limited to this example. Furthermore, the term "at least one of" if used to associate a list, such as A, B, or C, can be interpreted to mean any combination of A, B, and/or C, such as A, AB, AC, BC, AA, ABC, AAB, AABBCCC, etc.

Further, while certain embodiments have been described using a particular combination of hardware and software, it should be recognized that other combinations of hardware and software are also possible. Certain embodiments may be implemented only in hardware, or only in software, or using combinations thereof. In one example, software may be implemented with a computer program product containing computer program code or instructions executable by one or more processors for performing any or all of the steps, operations, or processes described in this disclosure, where the computer program may be stored on a non-transitory computer readable medium. The various processes described herein can be implemented on the same processor or different processors in any combination.

Where devices, systems, components or modules are described as being configured to perform certain operations or functions, such configuration can be accomplished, for example, by designing electronic circuits to perform the operation, by programming programmable electronic circuits (such as microprocessors) to perform the operation such as by executing computer instructions or code, or processors or cores programmed to execute code or instructions stored on a non-transitory memory medium, or any combination thereof. Processes can communicate using a variety of techniques, including, but not limited to, conventional techniques for inter-process communications, and different pairs of processes may use different techniques, or the same pair of processes may use different techniques at different times.

### Example Embodiments:

Embodiment 1: A method of determining physiological characteristics, the method comprising: obtaining an electromyography (EMG) signal representing muscle activity from an EMG electrode disposed in or on a band of a wrist-worn device, and a photoplethysmography (PPG) signal using a PPG sensor of the wrist-worn device; generating a modified PPG signal using the EMG signal, wherein the modified PPG signal corrects for motion artifacts in the PPG signal due to motion activity of a wearer of the wrist-worn device; and determining at least one physiological characteristic based on the modified PPG signal.

Embodiment 2: The method of embodiment 1, wherein the at least one physiological characteristic is at least one of: a heart rate of the wearer, a blood pressure of the wearer, or an oxygen saturation of the wearer.

Embodiment 3: The method of any one of embodiments 1 or 2, wherein the muscle activity comprises activity from at least one wrist extensor muscle or at least one wrist flexor muscle.

Embodiment 4: The method of any one of embodiments 1-3, wherein the motion activity of the wearer comprises finger movements.

Embodiment 5: The method of any one of embodiments 1-4, wherein an inertial measurement unit (IMU) of the wrist-worn device reports less motion activity than that associated with the EMG signal obtained.

Embodiment 6: The method of any one of embodiments 1-5, wherein the EMG electrode is disposed proximate to an artery of the wearer of the wrist-worn device, and wherein the artery is one of an ulnar artery or a radial artery.

Embodiment 7: The method of any one of embodiments 1-6, wherein more than two EMG electrodes are disposed in or on the band of the wrist-worn device.

Embodiment 8: The method of embodiment 7, further comprising selecting at least one EMG electrode of the more than two EMG electrodes from which to obtain an EMG for use in generating the modified PPG signal.

Embodiment 9: A method of determining physiological characteristics, the method comprising: obtaining an electromyography (EMG) signal representing muscle activity from an electromyography (EMG) electrode disposed in or on a band of a wrist-worn device, and a photoplethysmography (PPG) signal using a PPG sensor of the wrist-worn device; characterizing motion activity of a wearer of the wrist-worn device based on the EMG signal; and reporting a physiological characteristic determined using the PPG signal based at least in part on the characterization of the motion activity of the wearer.

Embodiment 10: The method of embodiment 9, wherein the physiological characteristic is at least one of: a heart rate of the wearer, a blood pressure of the wearer, or an oxygen saturation of the wearer.

Embodiment 11: The method of any one of embodiments 9 or 10, further comprising determining a manner in which the physiological characteristic is to be reported based on a type of physiological characteristic.

Embodiment 12: The method of any one of embodiments 9-11, wherein reporting of the physiological characteristic comprises at least one of: presenting an indication of the physiological characteristic with a warning, or inhibiting presentation of an indication of the physiological characteristic.

Embodiment 13: The method of any one of embodiments 9-12, wherein characterizing the motion activity comprises determining a type of motion activity.

Embodiment 14: The method of embodiment 13, further comprising comparing the PPG signal to an expected PPG signal selected based on the type of motion activity to form a comparison, wherein the reporting of the physiological characteristic is based on the comparison of the PPG signal to the expected PPG signal.

Embodiment 15: A wrist-worn device, the wrist-worn device comprising: at least one electromyography (EMG) electrode configured to generate EMG signals; at least one photoplethysmography (PPG) sensor configured to generate PPG signals; and at least one controller configured to: obtain an EMG signal representing muscle activity from the at least one EMG electrode disposed in or on a band of the wrist-worn device, and a PPG signal using the at least one PPG sensor of the wrist-worn device, generate a modified PPG signal using the EMG signal, wherein the modified PPG signal corrects for motion artifacts in the PPG signal due to motion activity of a wearer of the wrist-worn device, and determine at least one physiological characteristic based on the modified PPG signal.

Embodiment 16: The wrist-worn device of embodiment 15, wherein the at least one physiological characteristic is at least one of: a heart rate of the wearer, a blood pressure of the wearer, or an oxygen saturation of the wearer.

Embodiment 17: The wrist-worn device of any one of embodiments 15 or 16, wherein the muscle activity comprises activity from at least one wrist extensor muscle or at least one wrist flexor muscle.

Embodiment 18: The wrist-worn device of any one of embodiments 15-17, wherein the motion activity of the wearer comprises finger movements.

Embodiment 19: The wrist-worn device of any one of embodiments 15-18, wherein an inertial measurement unit (IMU) of the wrist-worn device reports less motion activity than that associated with the EMG signal obtained.

Embodiment 20: The wrist-worn device of any one of embodiments 15-19, wherein the EMG electrode is disposed proximate to an artery of the wearer of the wrist-worn device, and wherein the artery is one of an ulnar artery or a radial artery.

The specification and drawings are, accordingly, to be regarded in an illustrative rather than a restrictive sense. It will, however, be evident that additions, subtractions, deletions, and other modifications and changes may be made thereunto without departing from the broader scope as set forth in the claims. Thus, although specific embodiments have been described, these are not intended to be limiting. Various modifications and equivalents are within the scope of the following claims.

## Claims

1. A method of determining physiological characteristics, the method
comprising:
obtaining an electromyography (EMG) signal representing muscle activity from an EMG electrode disposed in or on a band of a wrist-worn device, and a photoplethysmography (PPG) signal using a PPG sensor of the wrist-worn device;
generating a modified PPG signal using the EMG signal, wherein the modified PPG signal corrects for motion artifacts in the PPG signal due to motion activity of a wearer of the wrist-worn device; and
determining at least one physiological characteristic based on the modified PPG signal.

2. The method of claim 1, wherein the at least one physiological characteristic is at least one of: a heart rate of the wearer, a blood pressure of the wearer, or an oxygen saturation of the wearer.

3. The method of claim 1, and any one or more of:
a) wherein the muscle activity comprises activity from at least one wrist extensor muscle or at least one wrist flexor muscle; or
b) wherein the motion activity of the wearer comprises finger movements.

4. The method of any preceding claim, wherein an inertial measurement unit (IMU) of the wrist-worn device reports less motion activity than that associated with the EMG signal obtained.

5. The method of any preceding claim, wherein the EMG electrode is disposed proximate to an artery of the wearer of the wrist-worn device, and wherein the artery is one of an ulnar artery or a radial artery.

6. The method of any preceding claim, wherein more than two EMG electrodes are disposed in or on the band of the wrist-worn device, in which case optionally further comprising selecting at least one EMG electrode of the more than two EMG electrodes from which to obtain an EMG for use in generating the modified PPG signal.

7. A method of determining physiological characteristics, the method comprising:
obtaining an electromyography (EMG) signal representing muscle activity from an electromyography (EMG) electrode disposed in or on a band of a wrist-worn device, and a photoplethysmography (PPG) signal using a PPG sensor of the wrist-worn device;
characterizing motion activity of a wearer of the wrist-worn device based on the EMG signal; and
reporting a physiological characteristic determined using the PPG signal based at least in part on the characterization of the motion activity of the wearer.

8. The method of claim 7, and any one or more of:
a) wherein the physiological characteristic is at least one of: a heart rate of the wearer, a blood pressure of the wearer, or an oxygen saturation of the wearer; or
b) further comprising determining a manner in which the physiological characteristic is to be reported based on a type of physiological characteristic; or
c) wherein reporting of the physiological characteristic comprises at least one of: presenting an indication of the physiological characteristic with a warning, or inhibiting presentation of an indication of the physiological characteristic.

9. The method of claim 7 or 8, wherein characterizing the motion activity comprises determining a type of motion activity, in which case optionally further comprising comparing the PPG signal to an expected PPG signal selected based on the type of motion activity to form a comparison, wherein the reporting of the physiological characteristic is based on the comparison of the PPG signal to the expected PPG signal.

10. A wrist-worn device, the wrist-worn device comprising:
at least one electromyography (EMG) electrode configured to generate EMG signals;
at least one photoplethysmography (PPG) sensor configured to generate PPG signals; and
at least one controller configured to:
obtain an EMG signal representing muscle activity from the at least one EMG electrode disposed in or on a band of the wrist-worn device, and a PPG signal using the at least one PPG sensor of the wrist-worn device,
generate a modified PPG signal using the EMG signal, wherein the modified PPG signal corrects for motion artifacts in the PPG signal due to motion activity of a wearer of the wrist-worn device, and
determine at least one physiological characteristic based on the modified PPG signal.

11. The wrist-worn device of claim 10, wherein the at least one physiological characteristic is at least one of: a heart rate of the wearer, a blood pressure of the wearer, or an oxygen saturation of the wearer.

12. The wrist-worn device of claim 10 or 11, wherein the muscle activity comprises activity from at least one wrist extensor muscle or at least one wrist flexor muscle.

13. The wrist-worn device of any one of claims 10 to 12, wherein the motion activity of the wearer comprises finger movements.

14. The wrist-worn device of any one of claims 10 to 13, wherein an inertial measurement unit (IMU) of the wrist-worn device reports less motion activity than that associated with the EMG signal obtained.

15. The wrist-worn device of any one of claims 10 to 14, wherein the EMG electrode is disposed proximate to an artery of the wearer of the wrist-worn device, and wherein the artery is one of an ulnar artery or a radial artery.
